(19) Europäisches Patentamt

European Patent Office

Office européen des brevets

(11) **EP 1 458 290 B1**

(12) **EUROPEAN PATENT SPECIFICATION**

(45) Date of publication and mention
of the grant of the patent:
**29.04.2009 Bulletin 2009/18**

(21) Application number: **02798385.7**

(22) Date of filing: **27.12.2002**

(51) Int Cl.:
*A61B 5/11* (2006.01)     *A61B 5/02* (2006.01)

(86) International application number:
**PCT/NO2002/000498**

(87) International publication number:
**WO 2003/061473 (31.07.2003 Gazette 2003/31)**

(54) **USE OF SENSOR AND SYSTEM FOR MONITORING HEART MOVEMENTS**

VERWENDUNG EINES SENSORS UND SYSTEMS ZUR ÜBERWACHUNG DER
HERZBEWEGUNGEN

UTILISATION D'UN DETECTEUR ET D'UN SYSTEME DE SURVEILLANCE DES MOUVEMENTS
DU COEUR

(84) Designated Contracting States:
**AT BE BG CH CY CZ DE DK EE ES FI FR GB GR
IE IT LI LU MC NL PT SE SI SK TR**

(30) Priority: **27.12.2001 NO 20016385**

(43) Date of publication of application:
**22.09.2004 Bulletin 2004/39**

(73) Proprietor: **Bio Medisinsk Innovasjon AS
0349 Oslo (NO)**

(72) Inventors:
• **ELLE, Ole, Jacob
N-0475 Oslo (NO)**
• **FOSSE, Erik
N-0458 Oslo (NO)**
• **GULBRANDSEN, Martin, G.
N-0674 Oslo (NO)**

(74) Representative: **Plougmann & Vingtoft A/S
Sundkrogsgade 9
P.O. Box 831
2100 Copenhagen Ø (DK)**

(56) References cited:
**WO-A-95/33517          WO-A-97/25099
US-A- 4 204 544          US-A- 5 109 842
US-A- 5 161 540          US-A- 5 628 777**

• **RICKARDS A.F. ET AL.: 'An implantable
intracardiac accelerometer for monitoring
myocardial contractility' PACE vol. 19, no. PART
I, December 1996, pages 2066 - 2071,
XP002963819**
• **WOOD JOHN C. ET AL.: 'Time-frequency
transforms: a new approach to first heart sound
frequency dynamics' IEEE TRANSACTIONS ON
BIOMEDICAL ENGINEERING vol. 39, no. 7, July
1992, XP000294410**

**Description**

When the blood supply in a cardiac vessel is obstructed (e.g. by ischemia, i.e. when the heart muscle does not receive sufficient oxygen) the muscles supplied by this artery will initiate an anaerobic metabolism and gradually lose contractility, which in turn results in a reduced heart activity (pumping effect). Often, surgical intervention is required in patient whose supply of blood is thus obstructed. The state of the patient's heart can be measured and monitored in several ways before, during and after an operation. The most common measurement of ischemic heart disease and reduced heart activity is ECG, systolic blood pressure (low sensitivity), central venous oxygen saturation through a Swan Ganz catheter (high sensitivity) and measurements of the heart activity through a Swan Ganz catheter.

Following heart surgery, for instance for angina pectoris, where a graft - a new vein - is laid past the occluded point in the coronary artery (a "bypass operation, it is of vital importance that the activity be monitored during the first few days following surgery. During the initial phase after the operation, occlusion of a graft surgically inserted into a small cardiac vessel is a risk to the patient. Such an occlusion does not necessarily present any immediate haemodynamic signs. A direct measurement of the movements in the muscle supplied by the target artery may however provide a higher sensitivity and earlier information regarding dysfunction.

A sensor system that can easily measure the movements of the heart muscles following heart surgery, particularly after revascularisation of the heart, would be an excellent monitoring tool for early warning and indication for re-intervention.

There are several types of measuring devices that may be used to measure the movement of the heart muscle. One method used to evaluate the heart movement is ultra sound imaging. However, this is relatively unsuitable due to the fact that such monitoring would require manual guiding of the ultra sound transducer. CT and MR are not suitable for post-operative monitoring a patient over a period of time, among other things because the use of such equipment would be very expensive.

WO 95/33517 relates to apparatus for monitoring_cardiac contractility. A triaxial motion sensor is mounted on the tip of a catheter inserted into the ventricle of the heart. By contacting the interior ventricular wall, a signal responsive to the natural heart acceleration can be provided.

US 5,628,777 relates to implantable leads or patch electrodes incorporating accelerometer-based cardiac wall motion sensors, and a method of fabricating such leads. The sensors transduce three-dimensional accelerations of cardiac tissue to provide electrical signals indicative of cardiac wall motion to an implantable cardiac stimulating device that uses the signal to detect and discriminate among potentially malignant cardiac arrhythmias. In response to a detected abnormal cardiac rhythm, the cardiac stimulating device may deliver therapeutic electrical stimulation to selected regions of cardiac tissue.

It has now been found that an efficient way of monitoring the state of a patient's heart after an operation is to use a light sensor system that may be fitted to the surface or immediately below the outer surface of the heart muscle, and which emits signals that reflect the movements of the heart. This may be one or possibly more sensors arranged in a pattern in order to monitor a heart surface, and may be one of the following types :

   1. Inertia-based elements such as accelerometers and gyroscopes based on: Capacitive sensor Piezo-resistive sensor Piezoelectric sensor

   2. Resonating accelerometers

   3. Thermal accelerometers

   4. Electro-mechanical sensors (position sensors, gyroscopes, potentiometers).

   5. Magnetic sensor elements

   6. Acoustic sensor elements

   7. Optical sensor elements.

   8. Bio-sensors (to register e. g. pCOz instead of movement)

   9. Combinations of the above. As an example, bio-sensors may be integrated into an electrode together with an accelerometer.

Moreover, it is known to equip a pacemaker electrode with an accelerometer for permanent implantation into the heart in order to detect irregularities in the heart rhythm, and which starts the pacemaker when such irregularities occur, cf. US patents no. 4 428 378, no. 4 140 132 and no. 5 833 713. Such a pacemaker device however, is not suited for such use as mentioned above, as this is placed in the apex inside the right or left ventricle. Prior art detects arrhythmia but will not be able to measure changes in the contractility of the heart muscle in specific areas as a function of the blood supply.

Thus one of the objects of the invention is to make a sensor element small enough to allow it to be placed underneath the membrane in the heart surface (epicardium) of a patient in the same manner as temporary pacemaker electrodes that today are routinely implanted during any bypass operations on the heart, as described in e.g. WO 97/25099. A pattern of sensors will make it possible to monitor the surface movements of the heart during the convalescence period, after which it can be withdrawn without requiring surgical intervention. Optionally, the sensor may be integrated into the pacemaker electrode, to allow the same electrode to be used both for measuring movements and pacing the heart when required.

The sensor is placed on the surface in the area that is supplied by the vessel that has been revascularised, e. g. apex in the case of revascularisation of the LAD (Left anterior descending), as the risk of compaction is at its greatest in the vessels which have undergone surgery. Preferably, the sensor is fastened by being placed between the epicardium and the myocardium in the same way as that used today to fasten temporary pacemaker electrodes..

A preferred concept is based on a capacitive accelerometer that can measure low frequencies down to 0 Hz and with large amplitudes. However, this is larger and heavier than the piezoelectric and piezo-resistive units, but is based on transport of very low energy. This will be crucial in said application. Placing the electronics in the sensor in order to prevent noise from stray capacitance in the wires from affecting the signal to any significant degree, and using micro/nano electromechanical methods (MEMS/NEMS) of reduction, e. g. by laying thin structures on top of a silicone substrate (surface micro machining), will allow these to compete with the above both in terms of size and characteristics.

Another preferred concept is based on the use of a piezoelectric accelerometer. Such accelerometers can be very small and light if based on thin films of piezoelectric material laid on a supporting structure. Such a sensor will also manage with only two conductors (wires), which is an advantage.

Another preferred concept is based on a piezo-resistive accelerometer, which can also be based on surface micromachining of e. g. a silicone substrate. As the piezo-resistive principle is not as sensitive to surrounding electrical noise, such as that induced via the wires to and from the sensors, this will allow the construction of a very small sensor with the electronics placed externally.

A method based on Bulk Micromachining will, with reduction (miniaturisation), also be a good alternative for this.

Thermal accelerometers based on convection have a good combination of sensitivity and overload protection.

Resonating sensors, both accelerometers and gyro sensors, are relatively complex, and in the case of gyro sensors, they are based on Coriolis acceleration. These are high precision sensors.

The above is provided with a device of the type mentioned by way of introduction, the characteristics of which appear from the independent claims. Further characteristics of the invention appear from the remaining, dependent claims.

In the following, the invention will be described in greater detail with reference to the drawing, in which:

| | |
|---|---|
| Fig. 1a | shows a detail of a commercially available temporary pacemaker electrode fitted with a sensor such as a triaxial accelerometer, |
| Fig. 1b | shows the entire pacemaker electrode of Fig. 1a; |
| Figs. 2a -b | shows the acceleration before and after occlusion; |
| Fig. 3 | shows a spectrogram of the acceleration signal throughout the course of events during artificially induced occlusion and ischemia; |
| Figs. 4a-4c | shows the frequency distribution calculated by means of a fast Fourier transform; |
| Fig. 5 | is a curve showing the energy ($X_i$) over time for a specific frequency on Fig. 3; |
| Figs. 6-12 | illustrate the signal processing in example 2 below; |
| Figs. 13 - 14 | illustrate the placement of the sensor on an active heart; |
| Figs. 15 - 16 | illustrate the connection of the sensor to a larger system. |

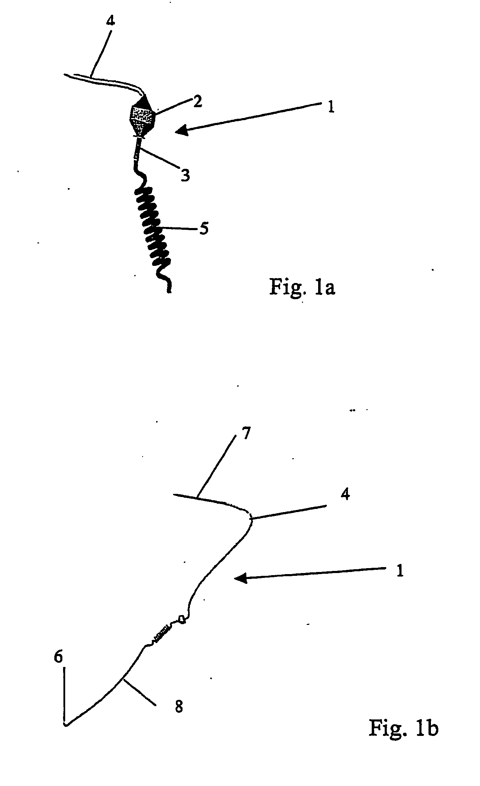

Fig. 1 illustrates a preferred embodiment of the invention with a commonly known temporary pacemaker electrode for use in the present invention. Reference number 2 denotes the accelerometer sensor arranged immediately above the conductive pacemaker electrode 3. It is envisaged that this 3-axis sensor 2 in particular be developed with micro-electro-mechanical or possibly nano-electro-mechanical methods (MEMS/NEMS) in order to make it small enough for such special purposes. Reference number 4 denotes insulated conductors to the pacemaker for connecting this to a pacemaker machine externally of the patient, and the wire 5 is the connecting wire. At the end of wire 5 is a hook shaped needle 6 for placing the pacemaker electrode in myocardium, and the end of the wire 4 is provided with a straight needle to allow the wire to be passed through the patient's thorax to the pacemaker machine. The accelerometer sensor 2 furthermore has a shape, indicated in Fig. 1, which gives good contact with the heart muscle and at the same time allows it to be withdrawn from the heart muscle without damaging tissue etc. The wire 5 has been given the form of a spring, and upon insertion, this is tightened to provide good contact between the accelerometer sensor 2 and the heart muscle. Following insertion, the hook shaped needle 6 is cut, so that upon subsequent removal, the wire can be pulled out of the thorax area together with the accelerometer sensor. -

However, the construction of the sensor is not dependent on the senor also being equipped with a temporary pacemaker sensor. Other similar constructions of the sensor are also possible. On the whole, such a construction will generally be the same as that used for different temporary pacemaker electrodes.

A sensor for use in the present invention must meet the following requirements:

1. Small; meaning a size similar to that of the conducting nipple of a temporary pacemaker electrode.
2. Light so as not to interfere with the proper motion of the heart (small pressure forces).
3. Sensitive to low frequencies, e.g. frequencies in the area 0<f<200 Hz, i.e. a frequency range that registers the various frequency components of which the heart movement consists.
4. Sensitive to amplitudes in the voltage range 0<V<2.5V, with a sensitivity of more than 600mV/g
5. Low energy exchange in the sensor element. This is essential to ensure that the sensor element does not emit electrical pulses to interfere with the functioning of a heart.

Reading of the signal from the sensor(s) can be carried out both in the form of position, speed and acceleration. The method of reading will be dependent on the type of sensor selected. As an example, a magnetic sensor element is easily read with a magnetometer. A signal from an accelerometer is normally read as an electrical signal registered through wires connected to the sensor.

When reading a signal from an accelerometer, the voltage is read as a function of acceleration. A Fourier transform of this signal will be the preferred procedure used to monitor changes in the signal. A further description of the method of doing this is given in the examples below.

Example 1

Experiments have been carried out with two biaxial accelerometers (ADXL-202 carried by Analogue Devices in Norwood, MA, USA) mounted at 90° with respect to each other in order to form a triaxial collection of acceleration data. These sensors can give digital or analogue output signals that are proportional to the acceleration. In the experiment, sampling at a higher rate (1 kHz) was provided by use of the analogue output from the sensors. An operational amplifier was used for buffering the signals in order to render them more resistant to ambient noise, in particular to 50 Hz from the mains. The electronics and two accelerometers were placed in a small polymer box and sealed. Small holes were made in the base plate, which holes were used for attaching to the heart (sewing). The signals were processed as described below.

The above sensor was fastened with four sutures to the myocardium of the apex of a pig's heart. After a short period (5 min.) arterial pressure was applied centrally to the left anterior descending cardiac artery. Occlusion of the LAD (Left Anterior Descending) (after approx. 4 min) to create ischemia. After approximately 6 minutes, the heart is ischemic and fibrillating.

In principle, the speed and position of the sensor may be reconstructed by a single and double integration of the acceleration signal. However, this is not entirely correct, as the rolling of the sensor is not measured, but the main problem is the presence of noise, as integration of noise results in Brownian motion. This may conceivably be solved either by filtering out as much integration noise as possible or by constructing a parameterized model of the heart's acceleration, which is then fitted to the measurement data. Such a model may for instance be based on a truncated Fourier series. However, in order to detect only abnormal heart activity, it would be more advantageous to base further analyses directly on the acceleration signal. Such analysis is described in greater detail in the following.

The accelerometer is sampled at regular intervals. Each sample consists of three components, the acceleration in the x, y and z directions (relative to a system of coordinates linked to the actual accelerometer). If i denotes the time interval (i =1, 2,....) this gives three time sequences, called e.g. $x_i$, $y_i$ and $z_i$. Due to the weight, these time sequences will include a considerable offset, that is the average will not be 0 over time. As this component of the acceleration has no connection with the heart activity, this is undesirable. Therefore, a sliding average is removed from each time sequence, giving new time sequences $u_i$, $v_i$ and $w_i$. To be more precise,

$$u_i = \frac{1}{n+1} \sum_{k=0}^{n} x_{i-k}$$

and correspondingly for

$$v_i = \frac{1}{n+1} \sum_{k=0}^{n} y_{i-k}$$

$$w_i = \frac{1}{n+1} \sum_{k=0}^{n} z_{i-k}$$

Here n indicates the length of the window, which should be great enough to allow the heart to beat several times in the course of n samples.

Now ai denotes the length of the vector with components $u_i$, $v_i$ og $w_i$. This quantity will then measure the acceleration of the heart wall without taking into account the direction of the acceleration. The direction of the acceleration was expected to be approximately constant and normal to the heart wall, so that no information was lost through studying only the length (if a suitable sign is defined for $a_i$). This was found not to be the case; on the contrary, the direction of the acceleration varied in almost all directions. In the following, the analysis is nevertheless limited to the time sequence $a_i$, as a one dimensional time sequence is easier to process, and it turns out that enough information has been kept to allow abnormal heart activity to be detected at an early stage.

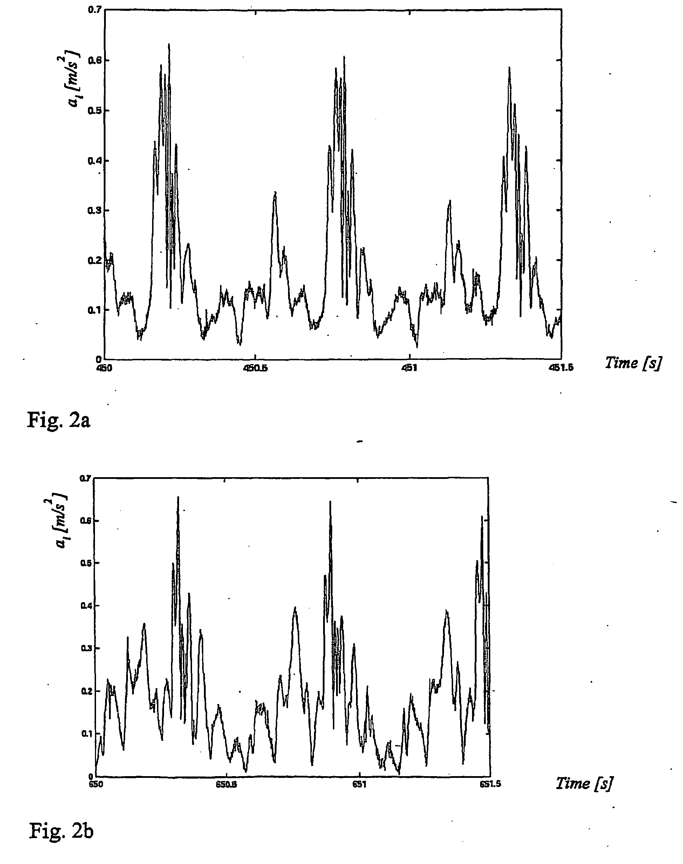

As an example, Figures 2a and 2b show the acceleration $a_i$ before and after occlusion in the previously mentioned experiment. One can see differences in the signal, but the nature of the change is difficult to establish without further signal processing.

The analysis of $a_i$ is based on a fast Fourier transform (FFT) that estimates the frequency distribution of a signal. Therefore the acceleration is sampled a certain number of times, for instance k times, to give a finite time sequence $a_1,\ldots,$ ak. Then FFT is applied to this sequence so as to give a new sequence $X_i$. Each element $X_i$ measures the energy of a frequency $f_i$ in the time sequence $a_i$. If T denotes the time between each sample, we find that $f_i = \frac{i}{2T}$

When studying the frequency image $X_i$ one will find big peaks at the frequency that corresponds to the pulse rate of the heart, and also the first multiples of this. Then if F denotes the pulse, frequency peaks will be found at the following frequencies: F, 2F, 3F,.... The Xi that correspond to different frequencies from these are approximately 0. In further analysis therefore, it is sufficient to consider the $X_i$ that correspond to these frequencies. So $A_1$ denotes the $X_i$ that corresponds to the pulse F, $A_2$ denotes the $X_i$ that corresponds to 2F etc. It is enough to look at a relatively small number of $A_i$, for instance the first 10 ($A_i$ approaches 0 when i increases). It has been observed that the numbers $A_i$ remain approximately constant for a healthy heart. If the heart is disturbed, the $A_i$'s change but return to the original pattern when normal heart activity is resumed. In experiments in which the LAD was blocked, a marked change could be observed in the $A_i$'s after a short time, long before fibrillation occurred. Based on this, we envisage that one set of $A_i$ can be determined, which applies to a normal, healthy heart, and then a subsequent sequence $A'_i$ can be compared with this standard, e.g. by calculating the sum of squares of the differences Ai - $A'_i$. This yields a single number that measures the deviation from normal activity.

An interesting question is whether there is a universal set of $A_i$ for a healthy heart or whether this is sensitive to the positioning of the accelerometer, whether there will be individual differences etc. New experiments will be able to determine this.

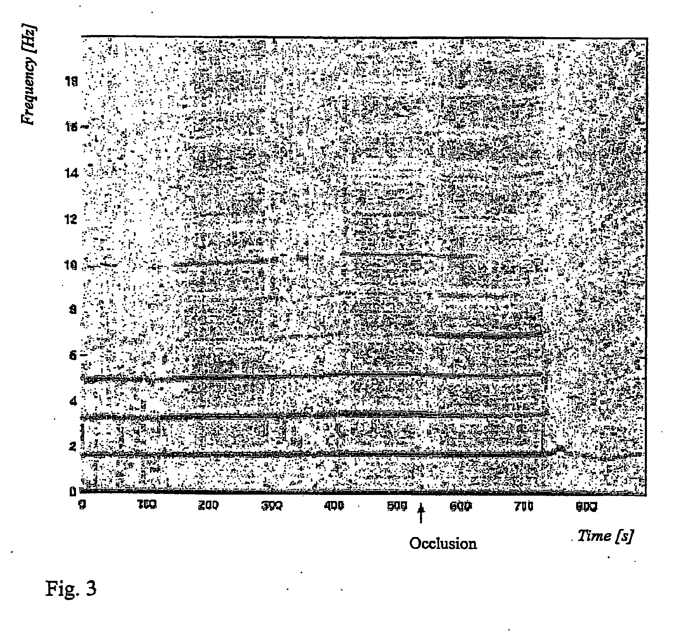

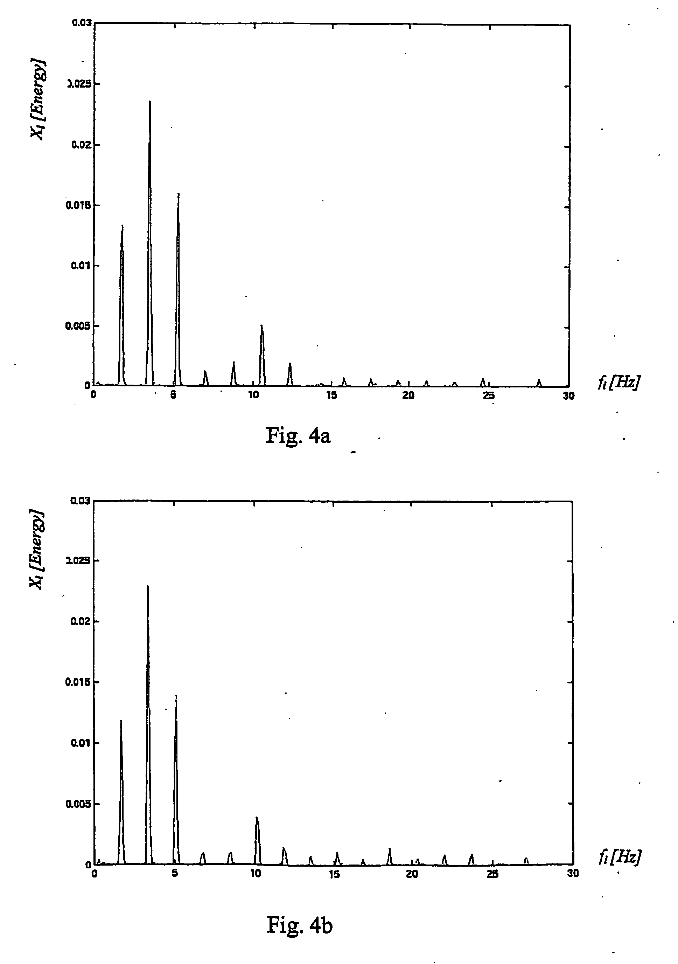

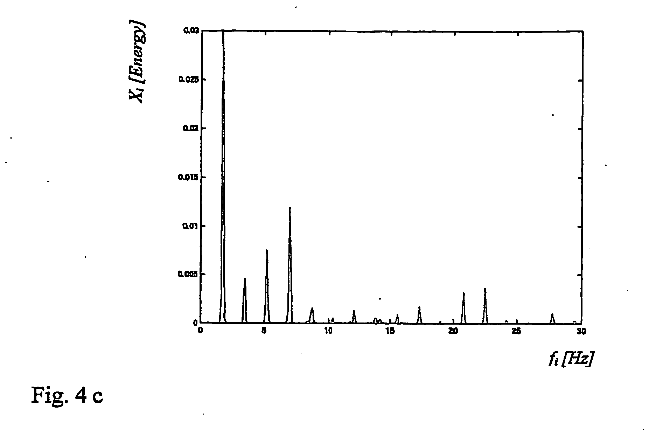

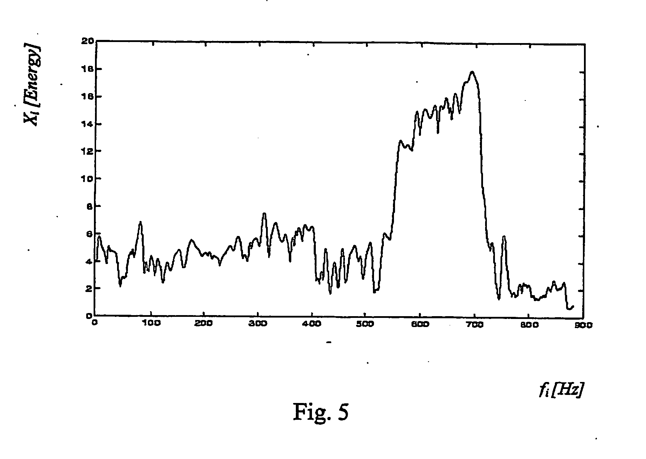

As an illustration of the above described principle, reference is made to Figure 3, which shows the development with time of the frequency distribution of the signal $a_i$, in the form of a spectrogram. Each vertical line is an approximation of the frequency distribution at a given point in time. In order to clarify the spectrogram, it may be shown in colours with the frequency distribution indicated e.g. on a scale of colour from blue via yellow to red as the strongest. The harmonies of the pulse $A_1$, $A_2$, ... will then appear as horizontal yellow lines. Upon occlusion, which takes place about 530-570 seconds after the start of the experiment, the intensity of the harmonies can be seen to change. This is shown more clearly in Figures 4a-c, which show the frequency distribution around the times 200, 450 and 650 seconds. Thus the first two are from before the occlusion and are almost identical. Figure 4c on the other hand, is from after the occlusion, and shows a marked deviation from the other two. Furthermore, Figure 5 shows, as an example, the energy $X_i$ that

corresponds to the fourth harmony $A_i$ of the pulse for a specific frequency (approx. 5 Hz) in Fig. 3 with time, the energy peak of the curve corresponding to red in the above mentioned scale of colours.

The diffuse band around 300 seconds is caused by the surgeon fastening the sutures in order to prepare for occlusion. It is worth noting that Figures 4a and 4b show the frequency distribution to be unaltered by this disturbance. This strengthens the hypothesis that a change in the frequency distribution indicates abnormal heart activity. During the experiment, occlusion sutures were placed in the myocardium around LAD (Left Anterior Descending) at 300-450 seconds after the initiation of the experiment, in order to prepare for occlusion. At 530-570 seconds, LAD is fully occluded, and the logging of the acceleration continues until the heart fibrillates. Figure 3 shows this to take place around 700 seconds after the start of the experiment, where the heartbeat at 1.7 Hz decreases (weaker curve). The first 150 seconds also show a blurred curve, which in all likelihood is caused by the heart becoming stressed during the fastening of the sensor.

The frequency distribution then provides an opportunity of detecting a difference in the acceleration before and after occlusion. The strength of the heartbeats that are harmonic to 1.7 Hz is altered after the occlusion. For instance, the fourth harmonic at 6-7 Hz is much stronger after the occlusion, while the sixth harmonic at approximately 10 Hz is weaker. This situation is shown more clearly in Figures 4a-c, which show the energy calculated by means of fast Fourier transform (FFT). Fig. 4a shows the calculations for the intervals of 200-210 seconds, Fig. 4b for the intervals of 450-460 seconds and Fig. 4c for the intervals of 650-660 seconds. It should be noted that Fig. 4a and Fig.4b are almost identical and similar to other intervals taken before the occlusion, as long as these are not within the disturbed area within which the implantation of the sensor takes place. After occlusion however, the image changes completely, cf. Fig.4c. As mentioned, Fig. 4c is shown for the interval 650-660 seconds, but the other intervals after the occlusion will display similar curves, though not as consistently identical as before the occlusion.

Changes in the frequency distribution can then be seen as a measurement of imminent ischemia, and this change can trigger an alarm for taking the required measures. I.e. when the frequency distribution dips below a predetermined value, the alarm is triggered. This value may be set e.g. with respect to the frequency distribution measured immediately after the insertion of the pacemaker electrode with the accelerometer.

In conclusion, a summary of the above described analysis:

1. Sample the accelerometer a number of times, remove offset by means of a sliding average and calculate the length $a_i$ of the acceleration vector.
2. Calculate the frequency distribution of the time sequence $a_i$ by means of FFT.
3. Based on the frequency distribution found, determine the pulse and let $A_1$, $A_2$,...be the energy of the first multiples of the pulse frequency.
4. Compare the set $A_i$ with a standard set for a healthy heart by calculating the sum of squares of the differences between $A_i$ and the standard set. A large sum of squares indicates abnormal heart activity.

Example 2

The object of this investigation was to detect the movement of the surface of the heart by means of a piezoelectric accelerometer for early warning in the case of ischemia during coronary surgery. The normal procedure for coronary bypass surgery was followed, CABG (Coronary Artery Bypass Grafting), LIMA-LAD with an open sternum. The accelerometer was fastened to the apex (left ventricle) for accelerometer measurements on occlusion of the LIMA and LAD respectively, in order to measure the changes in movement during ischemia (poor supply of blood to the heart muscle).

- Upon occlusion of the LAD the result of the anastomosis could be tested by looking at changes in the pattern of movements.
- Upon coincident occlusion of the LIMA the changes in movement during ischemia could be tested.

The details of what was to be investigated were as follows:

- How early can one detect ischemia by means of changes in the pattern of movements on 100% occlusion of the LAD, measured by means of accelerometers and analysed by means of fast Fourier transform (FFT) with continuous calculation of changes in the frequency spectra.?
- How sensitive is this detection to the placement of the sensor?
- How sensitive is the detection to the axial direction being measured?

  - Resultant of the x, y, z direction
  - One axis only

- What is the specificity of the system?

- What is the sensitivity of the system?

The accelerometer described in example 1 was fastened to the apex (left ventricle) of a pig where LIMA had been grafted to LAD through surgical intervention. LIMA and LAD were closed, and the effect on the signal from the accelerometer was detected in order to measure the changes in movement during ischemia (poor supply of blood to the heart) as described below. Upon occlusion of the LAD the functionality of the anastomosis will be tested by looking at changes in the pattern of movements. Upon coincident occlusion of the LIMA the changes in movement during ischemia will be tested.

The following was the procedure for logging of data:

1. The accelerometer was glued to the apex.
2. Measurements with installed accelerometer started after: a seconds.
3. LAD occluded to induce an ischemic condition after: b seconds.
4. Occlusion released to let heart recover, after: c seconds.
5. Logging of accelerometer data stopped.

Measuring : The acceleration along three orthogonal axes hereinafter termed the x, y and z directions, was measured using a newly developed 3D accelerometer. Analogue measurements were logged at 250 samples/s via Labview with DAQ 1200 Data Acquisition Card.

The log files are listed in table 1 below.

Below there follows a procedure for signal analysis of log data from log for animal testing 1:

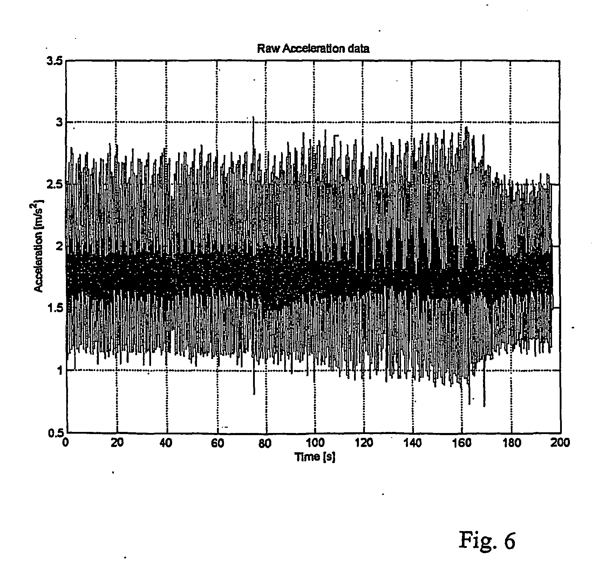

A 512 point fast Fourier transform (FFT) is used to analyse the raw data, illustrated in Figure 6, which shows unprocessed acceleration data in the x direction:

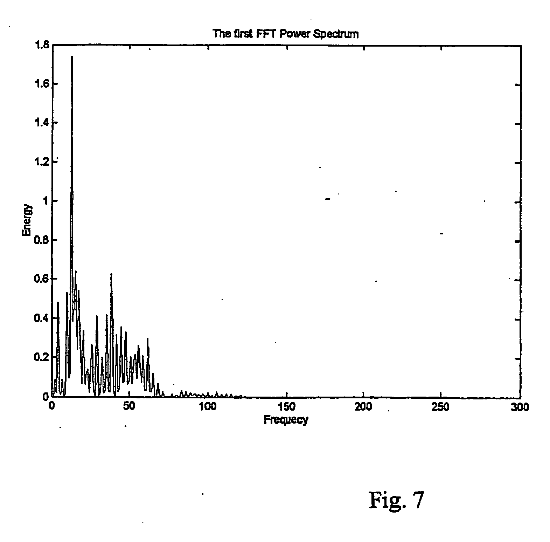

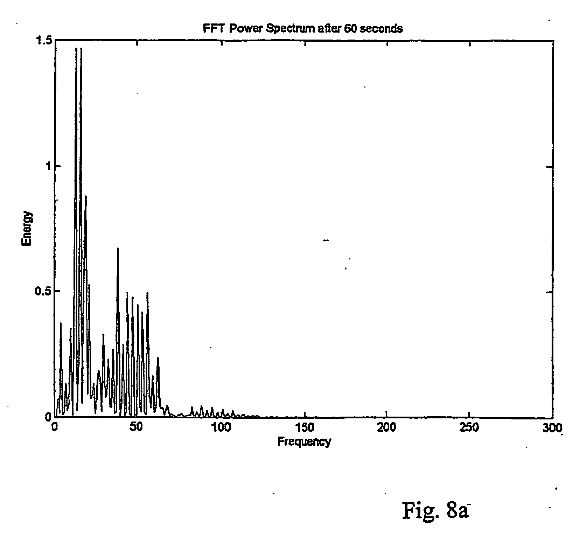

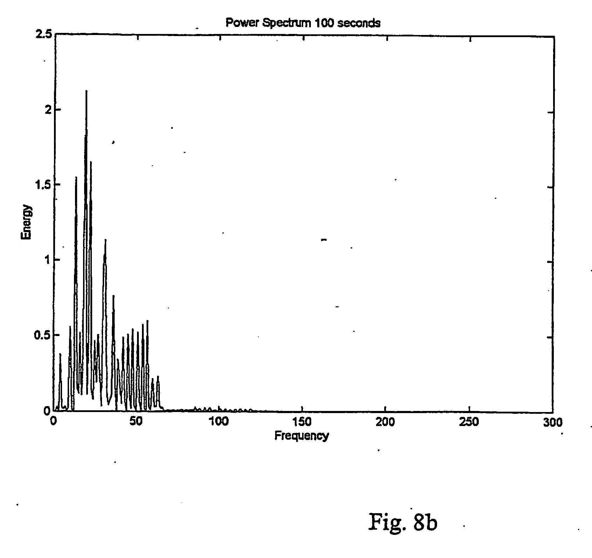

- Read acceleration measurement from the log file.
- Input the column, i.e. the axial direction, for which analysis was required, as a vector with a length equal to the log length.
- Took a 512 point subvector out of this (sample).
- Subtracted dc offset from the vector elements.
- Performed an FFT analysis with a 512 sample width, which resulted in a new vector but with complex elements.
- Calculated the "effect spectrum" by multiplying the FFT vector by its conjugate and then dividing by the length of the vectors. This provides a measure of energy as a function of frequency in the signal.
- Stored the first energy vector (see Figure 7). This vector can form a reference effect spectrum for the subsequent calculations.
- Then calculated the same energy vector with a floating window at increments that were a balance between the calculating speed and the requirement for accuracy/sensitivity, see Figures 8a and 8b, which show the energy spectrum after 60 and 100 seconds, respectively.
- The Euclidean distance between each of the new vectors and the first energy vector was calculated through the square root of the energies of each x value squared.
- Alarm as an indication of ischemia is triggered when:

  - The upper limit of the Euclidean distance (higher than the highest normal value found empirically) is exceeded.
  - The derivative of the same Euclidean distance exceeds a found upper limit.

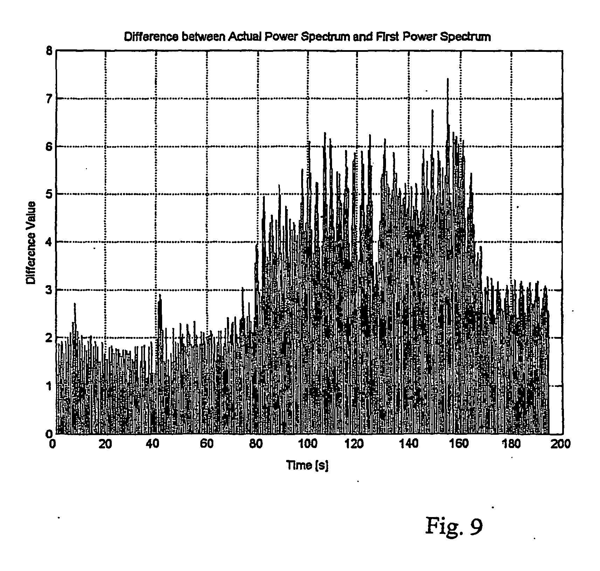

- Euclidean distance is plotted in real time, to allow a trained operator to see abnormal changes also from a graphical image. Figure 9 shows the development of the Euclidean distance from a floating effect spectrum to a reference spectrum (see Fig. 6), i.e. at the beginning of the measurements with the data from the floating window, so as to make it possible to see a change in the signal with respect to the first sampling, which then acts as a reference. Figure 9 clearly shows an increase in this differential value in the range 80 to 170 seconds

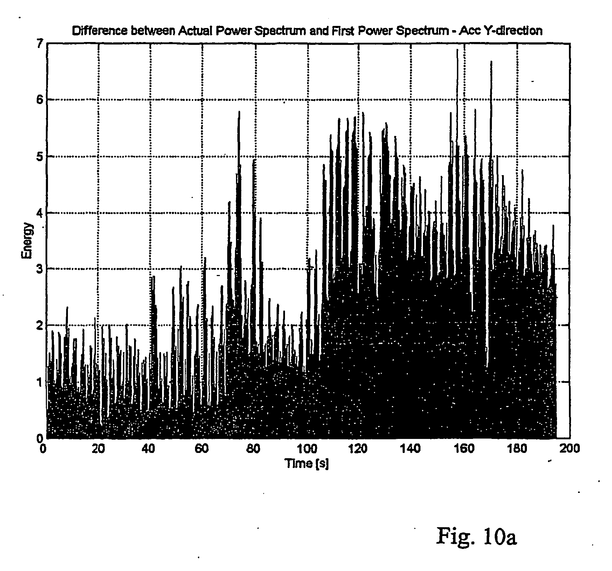

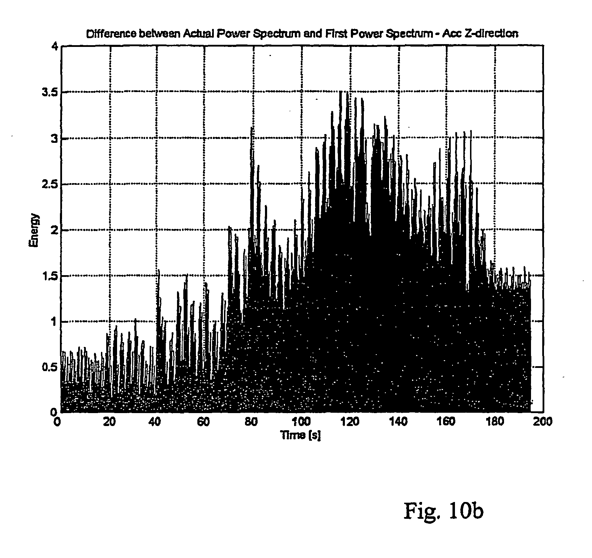

Figure 9 also shows the differential value for the spectrum along a first axis, called the x axis. In the test, the measurements were carried out using an accelerometer that is sensitive to movement along three axes, and Figures 10a and 10b show corresponding measurements performed along the y and z axes along the same time axis as for Figure 9.

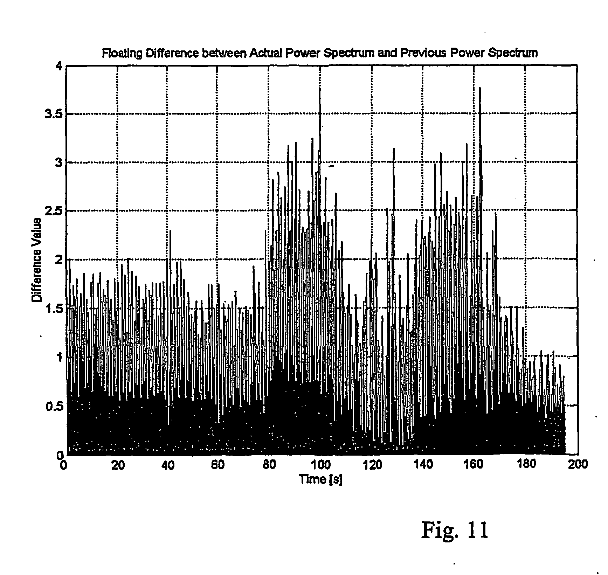

Figure 11 shows a signal difference between the last measured spectrum and the previous, so as to allow changes in the signal to be registered. As is apparent from comparison with Figure 9, the analysis method of Figure 11 provides a strong signal at the same points as those that show an increase in signal in Figure 9.

The experiments were carried out with two prototypes, where the signal processing of the signal from the first prototype was implemented into MatLab in the following way:

```
load pig250Hz_150802_apex1_80_85sec_35_lad.TXT;
x=pig250Hz_150802_apex1_80_85sec_35_lad(:,2);

x_window=x(1:512);
x_dc=x_window-mean(x_window);
fftx=fft(x_dc,512);
Pyx1 = fftx.* conj(fftx) / 512;
for i=2:50:(length(x)-512)
   x_window=x(i:511+i);
   x_dc=x_window-mean(x_window);
   fftx=fft(x_dc,512);
   Pxx = fftx.* conj(fftx) / 512;
   dist_eucl_x(i)=sqrt(sum((Pxx-Pxx1).*(Pxx-Pxx1)));
   i
end
figure
plot(dist_euc1_x);
```

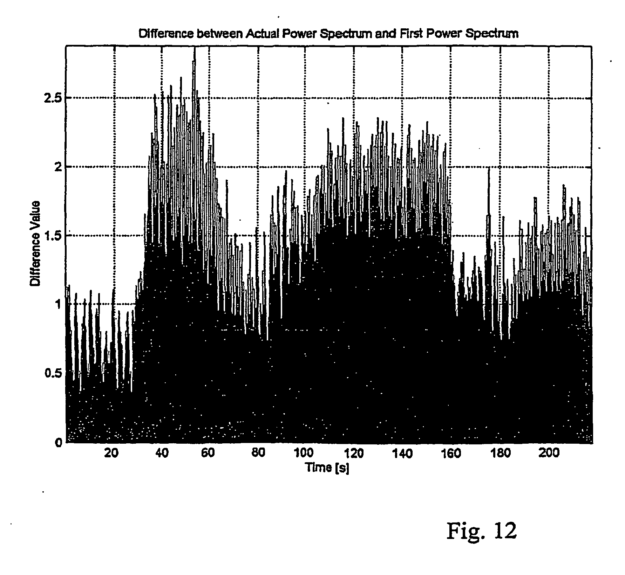

Figure 12 shows a result similar to that of Figure 9 for another experiment with another prototype, where the signal processing from the second prototype is implemented into MatLab in the following way:

```
load pig250Hz_150802_apex1_80_85sec_35_lad.TXT;
x=pig250Hz_150802_apex1_80_85sec_35_lad(:,2) ;

x_window=x(1:512);
x_dc=x_window-mean(x_window);
fftx=fft(x_dc,512);
Pxx_old = fftx.* conj(fftx) / 512;
for i=2:50:(length(x)-512)
   x_window=x(i:511+i);
   x_dc=x_window-mean(x_window);
   fftx=fft(x_dc,512);
   Pxx_new = fftx.* conj(fftx) / 512;
   dist_eucl_x(i)=sqrt(sum((Pxx_new-Pxx_old).*(Pxx_new-Pxx_old)));
   Pxx_old = Pxx_new;
   i
 end
 figure
 plot(dist_eucl_x);
```

As can be seen, the experiments show a surprisingly good correlation between the flow of blood in LAD (supply of blood to the left ventricle - apex) and changes in the accelerometer measurements. Proof of ischemia in apex can thereby be detected very early on, by accelerometer measurements being analysed in real time by means of fast Fourier transform (FFT) and calculations of Euclidean distance between the FFT spectra and the first FFT spectrum. A marked change in the Euclidean distance was detected almost immediately upon occlusion and opening respectively, of the LAD. (See Figure 9 under "animal testing 1", Acc X-direction, occlusion at 80 sec., reopened at 160 sec..

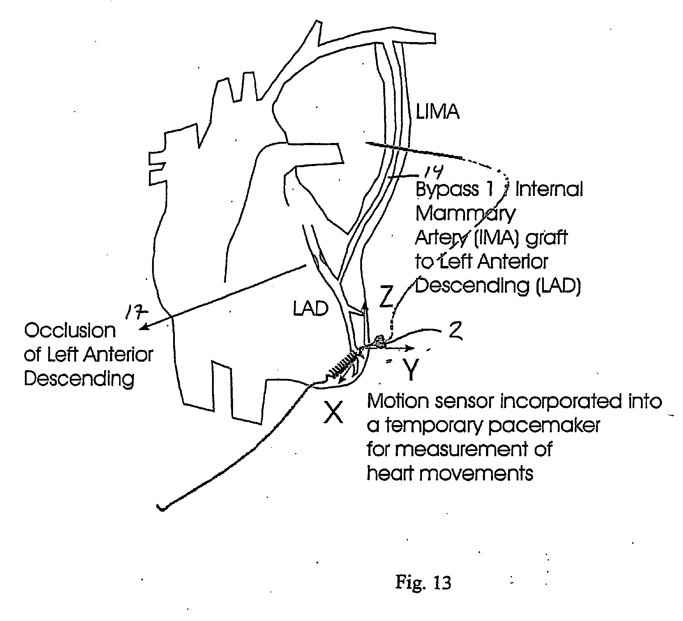

Figures 13-16 show the motion sensor in use on a heart that has been through a socalled bypass operation. As can be seen, the sensor is attached to a selected position on the surface of an active heart for registration of the movements of the heart in this position. According to a preferred embodiment of the invention, more, e.g. 2 or 3, sensors may be attached in different places on the heart. In this manner, post-operative monitoring of the heart after bypass surgery can be achieved. In order to achieve this, the motion sensor preferably has dimensions and fasteners designed to be removed from the position without requiring surgical intervention, e.g. dimensions such as those of a temporary pacemaker electrode.

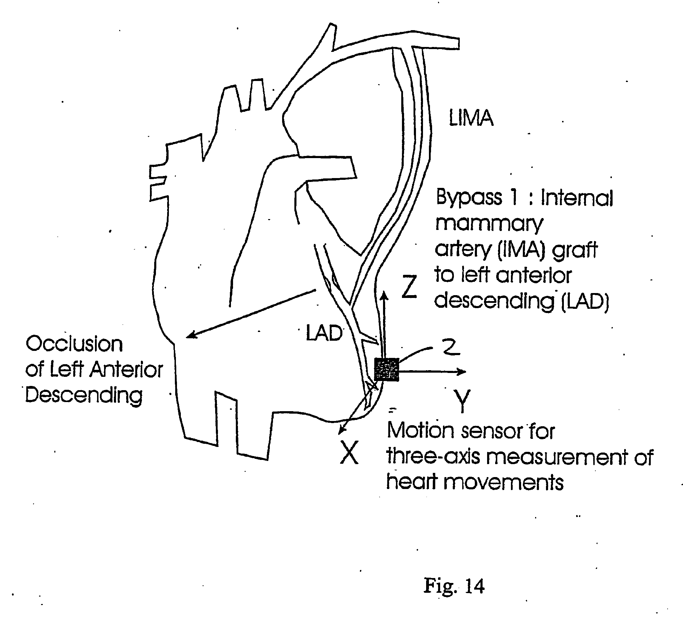

In the figures, a bypass is carried out past an area of reduced blood flow by reconnecting blood vessels 14 in order to supply blood to a specific area, and the sensor 2 is placed in an area where changes in the movements of the muscle caused by lack of blood supply, can be detected. As is apparent from Figure 14, the sensor is preferably sensitive in three directions.

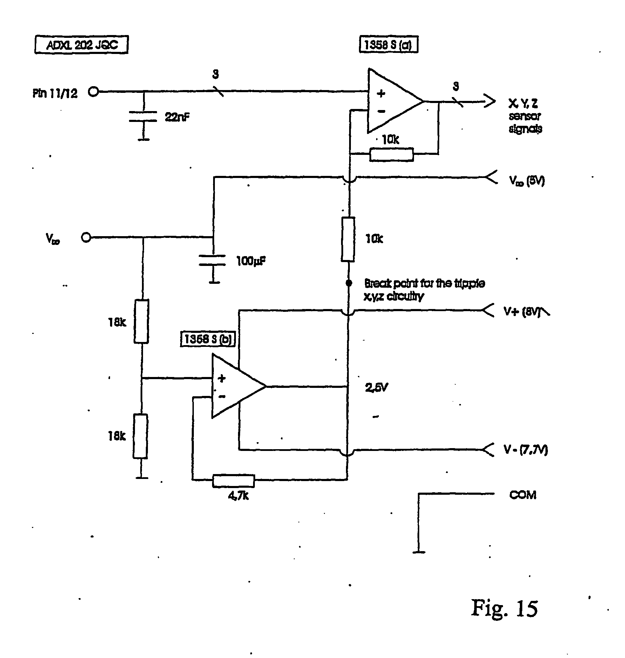

Figure 15 shows an example of a circuit diagram for the above acceleration sensor ADXL-202 used according to a preferred embodiment of the invention.

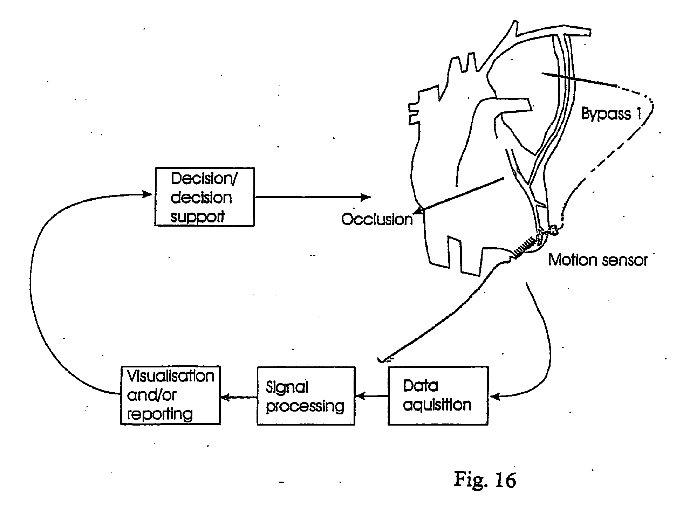

Figure 16 shows a sensor that is placed on the heart to carry out the measurements and is connected to a data acquisition unit 10, a unit 11 for signal processing 11 and further to a device 12 that displays the processed data on a screen and/or gives an acoustic warning in the case of deviations. The changes displayed or predicted can then form the basis 13 for deciding whether further surgical treatment is required or the patient can be pronounced fit.

Preferably, the position selected is a central point in that part of the heart muscle which after the operation is supplied with blood from the revascularised coronary artery.

Preferably, the motion sensor comprises an accelerometer that is sensitive to acceleration in at least one direction, but may as an alternative or supplement also comprise a gyroscope for measuring rotary movement in the locating point of the sensor. The gyroscope will be able to register other types of changes, e.g. if the selected position itself is at rest but the adjacent points are moving, causing twisting in that position.

The registered movement is transmitted to a calculation unit located externally of the patient, e.g. for Fourier analysis of the raw data from the sensor.

Preferably, the motion sensor according to the invention for registration of the movements of a heart will have a sensitivity of at least 600mV/g within a frequency range of 200Hz (band width) with a maximum amplitude of 2.5V. In order to allow it to be used in post-operative applications, its dimensions shall be smaller than 1.5x1.5x4 mm, preferably of the order of 1x1x2mm, and it is be provided with an outer material that does not cause reactions in biological materials, and devices for attaching it to a selected position on the surface of the heart, which sensor moreover comprises a signal conductor for transferring registered information to a calculation unit externally of the patient.

As mentioned, the motion sensor will have an accelerometer having three directions of sensitivity in order to register the direction of the movements.

The invention further comprises a system for monitoring changes in the movements of a heart muscle, such as shown in Figure 17, where the sensor is designed to emit signals that reflect the functioning of the heart, to a calculation unit. It may be tied in to further biosensors that are integrated into the accelerometer or fixed to the pacemaker electrode in order to give off signals that are characteristic to the functions of a heart.

The system further includes an amplifier and a calculation unit designed to amplify and calculate the signals, and a device for indicating the deviation upon comparison, e.g. by use of fast Fourier transform to determine the frequency distribution. The calculation unit determines the frequency distribution of the signals and compares this with a preset standard distribution, e.g. the first distribution measured immediately after the insertion of the sensor, such as in example 2 above. The system may further comprise a device for indicating the deviation from the predetermined values, which comprises an alarm transmitter designed to emit an alarm signal when the deviation from said standard distribution exceeds a certain level.

Table 1. Logging during two different animal tests:

| File name : | logging - base line [s] | occlusion [s] | reopening [s] | Comments (The times are relative) |
|---|---|---|---|---|
| pig_150802_apexl_80_85sec_35_lad.TXT | 80 | 85 | 35 | Log for 80s before occlusion for 85s before reopened for 35s (total log 200s) |
| pig_200802_apex1_30sec_128sek_60sec_lad.TXT | 30 | 128 | 60 | Log for 30s before occlusion for 128s before reopened for 60s (total log 218s) |

**Claims**

1. Method for analysing the movement of a selected position on the outer surface of a heart from an acceleration signal recorded by a motion sensor being designed by means of its dimensions to be removable from said selected position by pulling it out and fastened to, or immediately below, said selected position on the outer surface of the heart and registering the movements of the heart in this position in three directions, so that the acceleration signal describes the acceleration of said selected position in three dimensions over time, wherein said selected position is in an area of the heart that is supplied by a selected vessel, the method comprising analysing the recorded acceleration signal to calculate changes in movement of said selected position.

2. Method according to claim 1, wherein the method further comprises integrating the acceleration signal to reconstruct a speed and a position of said selected position on the surface of the heart.

3. Method according to any of the preceding claims, wherein the motion sensor is small enough to allow it to be fastened to, or immediately below, said selected position on the surface of the heart in that the motion sensor is based on thin films of piezoelectric material laid on a supporting structure.

4. Method according to any of the preceding claims, wherein the motion sensor has dimensions less than 1.5mm x 1.5mem x 4mm.

5. Method according to any of the preceding claims, wherein analysing the recorded acceleration signal comprises determining a frequency distribution of the recorded acceleration signal and comparing the determined frequency distribution with a reference frequency distribution recorded previously.

6. Method according to any of the preceding claims, wherein the motion sensor comprises a gyroscope for measuring rotary movement at the point of attachment of the sensor.

7. Method according to any of the preceding claims, wherein the registered movement is transmitted to a calculation unit located externally of the patient for performing said analysis.

8. Method according to any of the preceding claims, wherein the motion sensor is incorporated into a temporary pacemaker electrode.

9. A motion sensor (2) for registering the movements of a selected position on the outer surface of a heart, which sensor is a sensor with a sensitivity in three directions and is provided with external material that does not cause reactions in biological material and devices for fastening to, or immediately below, the selected position on the outer surface of the heart, which sensor furthermore comprises a signal conductor wire (4, 5) for transmitting registered information to a calculation unit located externally of the patient and by which the sensor can subsequently be removed by pulling it out, and which sensor has dimensions of less than 1.5mm x 1.5mm x 4mm.

10. A motion sensor according to claim 9 with a sensitivity of at least 600 mV/g within a frequency range of 200 Hz band width with a maximum amplitude of 2.5V.

11. A motion sensor according to claim 9, **characterised in that** the dimensions of the sensor are less than 1x1x2 mm.

12. The motion sensor according to claim 9, **characterised in that** the sensor is integrated into a temporary pacemaker electrode.

13. A motion sensor according to claim 9, **characterised in that** it comprises an accelerometer having three directions of sensitivity.

14. A system for detecting changes in the movement of the heart, e.g. related to ischemia, comprising at least one motion sensor according to claim 9 and a calculation unit, said calculation unit being adapted to analyze the registered movements and detecting changes in the pattern of the movements of the heart in the position of the sensor.

15. A system according to claim 14, **characterised in that** it further includes an amplifier and a calculation unit designed to amplify and calculate the signals, and a device for indicating deviation upon comparison.

16. A system according to claim 15, **characterised in that** the calculation unit uses fast Fourier transform for determining the frequency distribution.

17. A system according to claim 15, **characterised in that** the calculation unit determines the frequency distribution of the signals, and that these are compared with a pre-set standard distribution.

18. A system according to claim 15, **characterised in that** the pre-set standard distribution employed is the frequency distribution calculated immediately after insertion of the sensor.

19. A system according to claim 18, **characterised in that** it comprises a device for indicating deviation from predetermined values, comprising an alarm transmitter designed to emit an alarm signal when the deviation from said standard distribution exceeds a certain level.

20. A system according to claim 14, **characterised in that** the motion sensor is incorporated into a temporary pacemaker electrode.

**Patentansprüche**

1. Verfahren zum Analysieren der Bewegung einer ausgewählten Position an der Außenoberfläche eines Herzens auf der Grundlage eines Beschleunigungssignals, das von einem Bewegungssensor erfasst wird, der durch seine Dimensionen dafür eingerichtet ist, von der ausgewählten Position durch Ausziehen entfernt zu werden und an, oder unmittelbar unter, der ausgewählten Position an der Außenoberfläche des Herzens befestigt zu werden sowie Registrieren der Bewegungen des Herzens in dieser Position in drei Richtungen, damit das Beschleunigungssignal die Beschleunigung der ausgewählten Position in drei Richtungen im Zeitablauf beschreibt, wo die ausgewählte Position in einem Bereich des Herzens ist, der von einem ausgewählten Gefäß versorgt wird, wobei das Verfahren Analysieren des erfassten Beschleunigungssignals umfasst, um Bewegungsänderungen der ausgewählten Position zu berechnen.

2. Verfahren nach Anspruch 1, wo das Verfahren weiterhin Integrieren des Beschleunigungssignals umfasst, um eine Geschwindigkeit und eine Position der ausgewählten Position an der Oberfläche des Herzens wiederherzustellen.

3. Verfahren nach irgendeinem der vorhergehenden Ansprüche, wo der Bewegungssensor klein genug ist, um an, oder unmittelbar unter, der ausgewählten Position an der Oberfläche des Herzens befestigt werden zu können, indem der Bewegungssensor auf dünnen Folien aus einem piezoelektrischen Material, das auf einer Stützkonstruktion aufgelegt ist, basiert ist.

4. Verfahren nach irgendeinem der vorhergehenden Ansprüche, wo der Bewegungssensor Dimensionen von weniger als 1,5 mm x 1,5 mm x 4 mm aufweist.

5. Verfahren nach irgendeinem der vorhergehenden Ansprüche, wo das Analysieren des erfassten Beschleunigungssignals Bestimmen einer Frequenzverteilung des erfassten Beschleunigungssignals und Vergleichen der bestimmten Frequenzverteilung mit einer Referenzfrequenzverteilung, die vorher erfasst wurde, umfasst.

6. Verfahren nach irgendeinem der vorhergehenden Ansprüche, wo der Bewegungssensor ein Gyroskop zum Messen einer Rotationsbewegung am Befestigungspunkt des Sensors umfasst.

7. Verfahren nach irgendeinem der vorhergehenden Ansprüche, wo die registrierte Bewegung an eine Berechnungseinheit übermittelt wird, die außerhalb des Patienten zum Ausführen der Analyse angebracht ist.

8. Verfahren nach irgendeinem der vorhergehenden Ansprüche, wo der Bewegungssensor in eine vorläufige Herzschrittmacherelektrode eingebaut wird.

9. Bewegungssensor (2) zum Registrieren der Bewegungen einer ausgewählten Position an der Außenoberfläche eines Herzens, welcher Sensor ein Sensor mit einer Empfindlichkeit in drei Richtungen ist und mit Außenmaterial, das keine Reaktionen in biologischem Material verursacht, sowie Einrichtungen zum Befestigen an, oder unmittelbar unter, der ausgewählten Position an der Außenoberfläche des Herzens versehen ist, welcher Sensor weiterhin ein Signalleiterkabel (4, 5) zum Übermitteln registrierter Information an eine Berechnungseinheit umfasst, die außerhalb des Patienten angebracht ist und wodurch der Sensor anschließend durch Ausziehen entfernt werden kann, und welcher Sensor Dimensionen von weniger als 1,5 mm x 1,5 mm x 4 mm aufweist.

10. Bewegungssensor nach Anspruch 9 mit einer Empfindlichkeit von mindestens 600 mV/g im Frequenzbereich von 200 Hz (Bandbreite) mit einer maximalen Amplitude von 2,5V.

11. Bewegungssensor nach Anspruch 9, **dadurch gekennzeichnet, dass** die Dimensionen des Sensors kleiner als 1x1x2 mm sind.

12. Bewegungssensor nach Anspruch 9, **dadurch gekennzeichnet, dass** der Sensor in einer vorläufigen Herzschrittmacherelektrode eingebaut ist.

**13.** Bewegungssensor nach Anspruch 9, **dadurch gekennzeichnet, dass** er einen Beschleunigungsmesser mit drei Empfindlichkeitsrichtungen umfasst.

**14.** System zum Detektieren von Änderungen in der Bewegung des Herzens, z.B. in Verbindung mit Ischämie, umfassend mindestens einen Bewegungssensor nach Anspruch 9 und eine Berechnungseinheit, wobei die Berechnungseinheit dafür eingerichtet ist, die registrierten Bewegungen zu analysieren und Änderungen im Bewegungsmuster des Herzens in der Sensorposition zu detektieren.

**15.** System nach Anspruch 14, **dadurch gekennzeichnet, dass** es weiterhin einen Verstärker und eine Berechnungseinheit umfasst, die jeweils zum Verstärken und Berechnen der Signale eingerichtet sind, und eine Einrichtung zum Angeben von Abweichungen beim Vergleichen.

**16.** System nach Anspruch 15, **dadurch gekennzeichnet, dass** die Berechnungseinheit schnelle Fourier-Transformation zum Bestimmen der Frequenzverteilung anwendet.

**17.** System nach Anspruch 15, **dadurch gekennzeichnet, dass** die Berechnungseinheit die Frequenzverteilung der Signale bestimmt, und dass diese mit einer vorgegebenen Standardverteilung verglichen werden.

**18.** System nach Anspruch 15, **dadurch gekennzeichnet, dass** die verwendete vorgegebene Standardverteilung die Frequenzverteilung ist, die unmittelbar nach Einsetzen des Sensors berechnet wird.

**19.** System nach Anspruch 18, **dadurch gekennzeichnet, dass** es eine Einrichtung zum Angeben von Abweichungen von vorbestimmten Werten umfasst, umfassend einen Alarmübermittler, der dafür eingerichtet ist, ein Alarmsignal abzugeben, wenn die Abweichung von der Standardverteilung ein gewisses Niveau überschreitet.

**20.** System nach Anspruch 14, **dadurch gekennzeichnet, dass** der Bewegungssensor in einer vorläufigen Herzschrittmacherelektrode eingebaut ist.

**Revendications**

**1.** Procédé d'analyse du mouvement d'une position sélectionnée sur la surface externe d'un coeur à partir d'un signal d'accélération enregistré par un capteur de mouvement conçu au moyen de ses dimensions pour pouvoir être retiré de ladite position sélectionnée en le tirant et fixé sur, ou juste en dessous de ladite position sélectionnée sur la surface externe du coeur et enregistrant les mouvements du coeur dans cette position dans trois directions, de sorte que le signal d'accélération décrit l'accélération de ladite position sélectionnée en trois dimensions dans le temps, dans lequel ladite position sélectionnée est dans une zone du coeur qui est alimentée par un vaisseau sélectionné, le procédé comprenant l'analyse du signal d'accélération enregistré pour calculer les changements dans le mouvement de ladite position sélectionnée.

**2.** Procédé selon la revendication 1, dans lequel le procédé comprend en outre l'intégration du signal d'accélération pour reconstruire une vitesse et une position de ladite position sélectionnée sur la surface du coeur.

**3.** Procédé selon l'une quelconque des revendications précédentes, dans lequel le capteur de mouvement est suffisamment petit pour lui permettre d'être fixé sur, ou juste en dessous de ladite position sélectionnée sur la surface du coeur en ce que le capteur de mouvement est basé sur des films minces de matériau piézoélectrique posés sur une structure de support.

**4.** Procédé selon l'une quelconque des revendications précédentes, dans lequel le capteur de mouvement a des dimensions inférieures à 1,5 mm x 1,5 mm x 4 mm.

**5.** Procédé selon l'une quelconque des revendications précédentes, dans lequel l'analyse du signal d'accélération enregistré comprend la détermination d'une distribution de fréquence du signal d'accélération enregistré et la comparaison de la distribution de fréquence déterminée avec une distribution de fréquence de référence précédemment enregistrée.

**6.** Procédé selon l'une quelconque des revendications précédentes, dans lequel le capteur de mouvement comprend un gyroscope pour mesurer le mouvement de rotation au point de fixation du capteur.

**7.** Procédé selon l'une quelconque des revendications précédentes, dans lequel le mouvement enregistré est transmis à une unité de calcul située à l'extérieur du patient pour effectuer ladite analyse.

**8.** Procédé selon l'une quelconque des revendications précédentes, dans lequel le capteur de mouvement est intégré dans une électrode de stimulateur cardiaque temporaire.

**9.** Capteur de mouvement (2) pour enregistrer les mouvements d'une position sélectionnée sur la surface externe d'un coeur, lequel capteur est un capteur avec une sensibilité dans trois directions et est muni d'un matériau externe qui ne cause pas de réactions dans le matériel biologique et de dispositifs pour la fixation sur, ou juste en dessous de la position sélectionnée sur la surface externe du coeur, lequel capteur comprend en outre fil conducteur de signal (4, 5) pour transmettre les informations enregistrées à une unité de calcul située à l'extérieur du patient et grâce auquel le capteur peut ensuite être retiré en le tirant, et lequel capteur a des dimensions inférieures à 1,5 mm x 1,5 mm x 4 mm.

**10.** Capteur de mouvement selon la revendication 9, avec une sensibilité d'au moins 600 mV/g dans une gamme de fréquences de 200 Hz (largeur de bande) avec une amplitude maximale de 2,5 V.

**11.** Capteur de mouvement selon la revendication 9, **caractérisé en ce que** les dimensions du capteur sont inférieures à 1x1x2 mm.

**12.** Capteur de mouvement selon la revendication 9, **caractérisé en ce que** le capteur est intégré dans une électrode de stimulateur cardiaque temporaire.

**13.** Capteur de mouvement selon la revendication 9, **caractérisé en ce qu'**il comprend un accéléromètre à trois directions de sensibilité.

**14.** Système de détection des changements dans le mouvement du coeur, par exemple, liés à l'ischémie, comprenant au moins un capteur de mouvement selon la revendication 9 et une unité de calcul, ladite unité de calcul étant adaptée pour analyser les mouvements enregistrés et détectant les changements dans la structure des mouvements du coeur dans la position du capteur.

**15.** Système selon la revendication 14, **caractérisé en ce qu'**il comprend en outre un amplificateur et une unité de calcul destinés à amplifier et à calculer les signaux, et un dispositif pour indiquer l'écart lors d'une comparaison.

**16.** Système selon la revendication 15, **caractérisé en ce que** l'unité de calcul utilise la transformée de Fourier rapide pour déterminer la distribution de fréquence.

**17.** Système selon la revendication 15, **caractérisé en ce que** l'unité de calcul détermine la distribution de fréquence des signaux, et **en ce que** ceux-ci sont comparés avec une distribution standard prédéfinie.

**18.** Système selon la revendication 15, **caractérisé en ce que** la distribution standard prédéfinie employée est la distribution de fréquence calculée immédiatement après l'insertion du capteur.

**19.** Système selon la revendication 18, **caractérisé en ce qu'**il comprend un dispositif pour indiquer l'écart par rapport à des valeurs prédéterminées, comprenant un transmetteur d'alarme conçu pour émettre un signal d'alarme lorsque l'écart par rapport à ladite distribution standard dépasse un certain niveau.

**20.** Système selon la revendication 14, **caractérisé en ce que** le capteur de mouvement est intégré dans une électrode de stimulateur cardiaque temporaire.

Fig. 1a

Fig. 1b

Fig. 2a

Fig. 2b

Fig. 3

Fig. 4a

Fig. 4b

Fig. 4 c

Fig. 5

Fig. 6

Fig. 7

**FFT Power Spectrum after 60 seconds**

Fig. 8a

Fig. 8b

Fig. 9

Fig. 10a

Fig. 10b

Fig. 11

Difference between Actual Power Spectrum and First Power Spectrum

Fig. 12

LIMA

_14_
Bypass 1 / Internal
Mammary
Artery (IMA) graft
to Left Anterior
Descending (LAD)

Z

_17_

LAD

2

Occlusion
of Left Anterior
Descending

Y

X

Motion sensor incorporated into
a temporary pacemaker
for measurement of
heart movements

Fig. 13

LIMA

Bypass 1 : Internal
mammary
artery (IMA) graft
to left anterior
descending (LAD)

Z

LAD

Z

Occlusion
of Left Anterior
Descending

Y

X

Motion sensor for
three-axis measurement of
heart movements

Fig. 14

Fig. 15

Bypass 1

Decision/
decision
support

Occlusion

Motion sensor

Visualisation
and/or
reporting

Signal
processing

Data
aquisition

Fig. 16

**EP 1 458 290 B1**

**REFERENCES CITED IN THE DESCRIPTION**

**Patent documents cited in the description**

- WO 9533517 A
- US 5628777 A
- US 4428378 A
- US 4140132 A
- US 5833713 A
- WO 9725099 A